# EUROPÄISCHE PATENTANMELDUNG

(11) **EP 0 730 829 A2**
(43) Veröffentlichungstag der Anmeldung: **11.09.1996**
(21) Anmeldenummer: 95118123.9
(22) Anmeldetag: 17.11.1995
(51) Int. Cl.: A23J 1/00

(54) **Verfahren zur Gewinnung von Weisenstärke und/oder Weizenproteinhydrolysat**

(30) Priorität: 17.11.1994 DE 4440960; 17.11.1994 DE 4440958
(71) Anmelder: Sta Pro Consultancy B.V., NL- 9422 TH Smilde (NL)
(72) Erfinder: Lameijer, Engel Frans, ir., NL-9422 TH Smilde (NL); Tegtmeier, Uwe, Dr., D-31234 Edemissen-Rietze (DE)
(74) Vertreter: Gramm, Werner, Prof., Dipl.-Ing.

(57) **Zusammenfassung**

Die Erfindung betrifft ein Verfahren zur Gewinnung von Weizenstärke und/oder Weizenproteinhydrolysat. Die Ausgangsstoffe werden vermahlen und danach mit Wasser bei Temperaturen ≦ 50 °C zu einer Slurry verarbeitet, die durch Zentrifugieren getrennt wird in eine Stärke und Fasern enthaltende Fraktion und in eine glutenbildende Proteinfraktion. Vor oder nach dem Zentrifugieren werden der Slurry Enzyme zugegeben, um das glutenbildende und nicht glutenbildende Weizenprotein sowie weitere nicht protein- und nicht stärkehaltige Inhaltsstoffe in der Slurry zu einem Weizenproteinhydrolysat zu hydrolysieren. In die Mischung werden Scherkräfte eingetragen und zwar durch Umpumpen der Mischung durch ein Sieb, wobei den Feststoffanteilen der Mischung unmittelbar vor dem Sieb mittels einer eingesetzten Pumpe eine hohe Beschleunigung erteilt wird.

## Beschreibung

Die Erfindung betrifft ein Verfahren zur Gewinnung von Weizenstärke und/oder Weizenproteinhydrolysat.

Die Erfindung betrifft ferner eine Vorrichtung zur Durchführung dieses Verfahrens sowie die hergestellte Stärkemilch bzw. das hergestellte Proteinhydrolysat.

Während der vergangenen Jahre hat die Bedeutung der Modifizierung von Vitalkleber aus Weizen zugenommen. Vitaler Weizenkleber wird eingesetzt unter anderem für die Verbesserung der Backeigenschaften in Teigwaren; der modifizierte Weizenkleber findet überwiegend Anwendung bei der Ergänzung bzw. dem Ersatz von tierischem Protein und/oder pflanzlichem Protein im Futtermittelsektor, insbesondere für die Aufzucht von Jungtieren.

Der Vitalkleber wird während des Stärkeherstellungsprozesses aus Weizen gewonnen und hat üblicherweise ca. 78 % Proteinanteil. Er zeichnet sich durch eine weitgehende Wasserunlöslichkeit aus.

Die Herstellung eines modifizierten Weizenvitalklebers geschieht konventionell ausgehend von nassem Vitalkleber oder getrocknetem Vitalkleber, welche während des Stärkeherstellungsprozesses aus Weizen oder Weizenmehl gewonnen werden. Ausgehend von getrocknetem Vitalkleber wird dieser zuerst erneut mit Wasser angemischt. Die Temperatur der Mischung wird eingestellt in Abhängigkeit von der Optimumtemperatur der nachfolgend einzusetzenden Proteasen. Die entsprechenden Proteasen werden anschließend zugegeben bei gleichzeitiger Einstellung des pH-Wertes. Nach einer bestimmten Reaktionszeit werden die Enzyme durch Hitzedenaturierung mittels Anhebung der Temperatur und/oder durch Veränderung des pH-Wertes inaktiviert. Letztendlich wird das Produkt in einem Sprühtrockner getrocknet, wobei die Trockensubstanz mindestens 93 % mas betragen muß, um die Produktstabilität zu gewährleisten. Der Eiweißgehalt eines solchen Produktes beträgt ca. 80 % (berechnet auf der Basis des gemessenen Stickstoffes x Faktor 6,25). Die derzeitig hergestellten Mengen an modifiziertem Vitalkleber betragen - insbesondere durch die verstärkte Anwendung für die Herstellung von Futtermittel für die Jungtieraufzucht - mehr als 10.000 t/Jahr.

Der zuvor beschriebene Herstellungsprozeß ist ausführlich dargestellt auf Seite 4 der Patentoffenlegungsschrift Nr. WO 89/06091, Anmelde-Nr.: PCT/AU88/00497, Anmeldedatum: 23.12.1988.

Ein entscheidender Nachteil in dem bekannten Herstellungsprozeß ist, daß zuvor ein Vitalkleber bei der Herstellung von Weizenstärke in einem aufwendigen Prozeß abgetrennt und gereinigt werden muß.

Die Herstellung von Weizenstärke als Rohstoff für eine Weiterverarbeitung zu Endprodukten wie zum Beispiel Glucose hat in den vergangenen Jahren an Bedeutung gewonnen. Hiermit ist nicht nur eine erhöhte Weizenstärkeproduktion sondern auch eine Erhöhung der aus den konventionellen Verfahren resultierenden B-Stärkefraktion sowie des Weizenvitalklebers einhergegangen. Es wurden auch die aufnehmenden Märkte für den Weizenvitalkleber beeinflußt. Für den Einsatz in Futtermittel für die Jungtieraufzucht liegt die Hauptbedeutung des modifizierten Weizenklebers in seinem Eiweißgehalt und/oder in den funktionellen Eigenschaften begründet, welche durch die Modifizierung eingestellt werden können. Ein entscheidender Vorteil für die Verwendung des Weizens zur Stärkeherstellung ist der Gehalt an wertvollem Eiweiß im Weizen, welches ebenfalls gewonnen werden kann und zur Wirtschaftlichkeit der Stärkeherstellung am Weizen entscheidend beiträgt.

Nachteilig bei der Durchführung der konventionellen Technik für die Weizenstärkeherstellung ist insbesonders, daß die Ausbeute an qualitativ guter Stärke, der sogenannten A-Stärke, lediglich ca. 60 % auf Weizenmehl-TS beträgt.

Die Stärkeausbeute bei der Weizenstärkeherstellung wird entscheidend beeinflußt durch
- den Stärkegehalt im Weizen/Weizenmehl
- die Partikelgrößenverteilung der Weizenstärke
- den Eiweißgehalt des Weizens/Weizenmehles
- die Fähigkeit des wasserunlöslichen Proteins zu agglomerieren
- die Qualität des Eiweißes, d. h. Gewinnung des sogenannten Vitalklebers für den Backprozeß
- den Gehalt an Hemicellulose, Xylanose und Glucane sowie weiterer nichtstärke- und nichteiweißhaltiger Komponenten, der sich auf das Viskositätsverhalten des Weizenmehles in wässeriger Phase auswirkt
- die Wechselwirkungen zu den unterschiedlichen Komponenten
- das Verhältnis von wasserlöslichen und nichtwasserlöslichen Pentosanen und durch
- den Einfluß von im Prozeß eingesetzten Hilfsstoffen
Eine anwendbare Labormethode für die Ermittlung der Weizenmehlqualität mit Aussagen für die Produktion von Vitalkleber und Weizenstärke wurde beschrieben von P.L. Weegels et. all.: Small Scale Separation of Wheat Flour in Starch and Gluten, Starch/Stärke 40 (1988) Nr. 9, S. 342-346.

E. Lameijer et all.: Workshop over geluid, afvalwater, geur en energie in de Nederlandse zetmeelindustrie, 10. Mai 1990, Holiday Inn Hotel Utrecht, Organisator: Novem, Postbus 17, NL-6130 AA Sittard, vergleicht die unterschiedlichen Weizenstärkeherstellungsprozesse mit modernen analytischen Methoden. E. Lameijer et all. beschreibt die derzeit modernsten Weizenstärkeprozesse, den sogenannten Hydrozyklon-Prozeß und den 3-Phasen-Dekanter-Prozeß.

Wenn man diese modernen bekannten Prozesse betrachtet, so kann man feststellen, daß die bei der Weizenstärkeherstellung entstehenden Stärkeverluste vorrangig begründet sind durch
- Stärkeverluste in der dritten Phase des 3-Phasen-Dekanter-Prozesses, wobei die dritte Phase überwiegend die Pentosane enthält sowie Proteine und Stärke;
- Verluste im B-Stärke-Strom, der sowohl im 3-Phasen-Dekanter-Prozeß wie auch im Hydrozyklon-Prozeß anfällt, weil hiermit der Hauptanteil des Eiweißes abgetrennt wird;
- Stärkeverluste in Höhe von ca. 10 % mas auf TS im gewonnenen Vitalkleber; und durch
- Stärkeverluste bei der Abtrennung von Fasern und/oder unlöslichen Pentosanen (überwiegend im Hydrozyklon-Prozeß).

Angaben zu Verlusten und Ausbeuten im Weizenstärkeherstellungsprozeß finden sich ferner bei W. Kempf und C. Röhrmann, Detmold: Verfahren der industriellen Weizenstärkegewinnung auf Rohstoffbasis Weizenkorn, starch/Stärke 36 (1984) Nr. 1, S. 1 - 7, sowie bei B. I. Dahlberg, Tumba: A New Process for the Industrial Production Wheat Starch and Wheat Gluten, Starch/Stärke 30 (1978), Nr. 1, S. 8 - 12.

Die Erfindung beruht auf folgenden Überlegungen:
Während der letzten zehn Jahre ist der Einsatz von Enzymen in den ersten Prozeßschritten bei der Weizenstärkeherstellung in der einschlägigen Industrie vorgenommen worden. Die hier verwendeten Enzyme sind insbesondere Cellulasen, Hemicellulasen, Xylanasen, Glucanasen, etc., die ursächlich für eine Erniedrigung der Viskosität in der Slurry eingesetzt werden, die vor Durchführung der Trennschritte im Prozeß hergestellt wird. Diese Enzyme können jedoch nicht bestwirkend eingesetzt werden, da die Aufenthaltszeit für die Einwirkung begrenzt ist, und die prozeßbedingte Temperatur des Mediums nicht mit der spezifischen Optimumtemperatur der Enzyme und ferner auch der pH-Wert nicht mit den wirkungsspezifischen Optimum-Werten der Enzyme übereinstimmen.

Der Einsatz von Proteasen und/oder Peptidasen wird bislang in diesem Zusammenhang nicht durchgeführt, da durch den damit verbundenen Eiweißabbau die Ausbeute an hochwertigem Vitalkleber entsprechend reduziert wird.

Durch die Verwendungsmöglichkeit von modifiziertem Kleber zum Beispiel bei der Herstellung von Futtermittel für die Jungtieraufzucht eröffnen sich hier jedoch neue Perspektiven, indem die Modifizierung bereits in den Stärkeherstellungsprozeß ingegriert werden kann, und einhergehend eine Erhöhung der Stärkeausbeute möglich wird.

Der Erfindung liegt die Aufgabe zugrunde, die bekannten Verfahren zu vereinfachen und die damit hergestellten Produkte zu verbessern.

In der Weizenstärkeindustrie werden nach den heute angewandten Verfahren als Endprodukte A-Stärke, B-Stärke, C-Stärke sowie Weizenvitalkleber hergestellt. Ferner fällt ein Produktstrom an, der sich aus konzentriertem Prozeßwasser zusammensetzt, das überwiegend die löslichen und dispergierbaren Komponenten des Mehles enthält. Der kommerzielle Wert der Produkte B-Stärke, C-Stärke und konzentriertes Prozeßwasser ist sehr niedrig, insbesondere in Ländern, in denen diese Produktströme nicht direkt in flüssiger Form z. B. an Landwirte zur Verfütterung direkt abgegeben werden können.

Vor einigen Jahren wurde in dem üblichen Weizenstärkeherstellungsprozeß eine Verbesserung derart eingeführt, daß eine verkaufsfähige, sogenannte A⁻-Stärke, aus dem B-Stärke-Produkt zurückgewonnen wird. Diese A⁻-Stärke ist auch als "Small Granular Wheat Starch" (SGS) bekannt. Die A⁻-Stärke weist einen im Vergleich zur B-Stärke höheren Stärkegehalt auf, besitzt allerdings einen Proteingehalt von ca. 0,5 % auf Trockensubstanz, ist somit qualitativ minderwertiger als A-Stärke.

Diese Verbesserung führte zu einer Erhöhung der Ausbeute an qualitativ hochwertiger bzw. vermarktungsfähiger Stärke. Insbesondere die A⁻-Stärke kann entweder in Verbindung mit A-Stärke oder auch separat als Rohstoff für eine weitergehende Verarbeitung, z. B. zu Verzuckerungsprodukten wie Glucose und Fructose, eingesetzt werden.

Der Erfindung liegt die weitere Aufgabe zugrunde, die Stärkeausbeute weiter zu erhöhen und im Zusammenhang hiermit ein Weizenproteinhydrolysat zu gewinnen, das als Futtermittel eingesetzt werden kann. In dem Verfahren nach der Erfindung werden die A-Stärke, die A⁻-Stärke sowie gemäß einer Ausführungsform des Verfahrens auch Vitalkleber aus dem Weizenmehl abgetrennt und gereinigt, wobei der Rest der in dem Weizenmehl enthaltenen Komponenten insgesamt in einer proteinangereicherten Fraktion erhalten wird, die Weizenproteinhydrolysat genannt wird.

Aufgrund der Erzeugung von Weizenproteinhydrolysat wirken sich Preisschwankungen im Markt für Weizenvitalkleber nicht direkt auf die Gesamtwirtschaftlichkeit des Verfahrens aus.

Das Verfahren nach dieser Erfindung kann eingesetzt werden sowohl für die Errichtung neuer Weizenstärkeproduktionen als auch für die Verbesserung von bestehenden Produktionsstätten.

Das Verfahren nach dieser Erfindung betrifft mehrere Prozeßvarianten für die Produktion von A-Stärke, A⁻-Stärke, Vitalkleber und Weizenproteinhydrolysat.

Der Grundgedanke ist die Produktion von dispergierbaren Weizenproteinhydrolysat mit einem Proteingehalt, der im Bereich zwischen dem Proteingehalt des Weizenmehls und dem Proteingehalt des Vitalklebers oder enzymatisch bzw. chemisch durchgeführter Modifizierungen hiervon liegt.

Das Weizenproteinhydrolysat kann in der Futtermittelindustrie eingesetzt werden für die Herstellung von Kälbermilch, z. B. als Magermilchpulverersatz aufgrund seiner hervorragenden Verdaulichkeit sowohl bezüglich der Proteinfraktion als auch der enthaltenen Kohlehydrate. Antinutrielle Faktoren sind nicht enthalten. Es ist weiterhin möglich, durch Einführung von funktionellen Eigenschaften (z. B. Vernetzung und/oder Desamidierung) in das Weizenproteinhydrolysat auch eine Anwendbarkeit im Nahrungsmittelbereich zu erschließen.

Die eingangs gestellte Aufgabe wird hinsichtlich des Verfahrens erfindungsgemäß durch die Merkmale des Anspruchs 1 gelöst.

Erfindungsgemäß wird somit die enzymatische Behandlung des glutenbildenen und nichtglutenbildenden Proteins bereits in der Slurry vorgenommen, ohne daß eine Gewinnung von Vitalkleber mit vorhergehender Stärkeabtrennung notwendig ist.

Das erfindungsgemäße Verfahren läßt sich in vorteilhafter Weise so durchführen, daß das in den Reaktionsbereich zugegebene, eine Temperatur von < 60 °C, vorzugsweise 40 °C aufweisende Wasser gerührt und umgepumpt wird;
daß dem umgepumpten Wasser eine erste Teilmenge Weizenmehl zudosiert wird;
daß in die so gebildete Slurry Scherkräfte eingetragen werden;
daß nach der Enzymzugabe die Einstellung des pH-Wertes auf > 5,0 und < 6,0 erfolgt; und
daß unter Aufrechterhaltung der Scherkrafteintragung die Zudosierung der zweiten Weizenmehl-Teilmenge erfolgt.

Die vorstehend aufgelisteten Verfahrensschritte müssen nicht zwingend in der genannten Reihenfolge nacheinander durchgeführt werden.

Erfindungsgemäß ist es zweckmäßig, wenn für die Hydrolysierung und/oder Modifizierung des glutenbildenden und nichtglutenbildenden Proteins Proteasen und Peptidasen, für den Abbau von nicht protein- und nicht stärkehaltigen Inhaltsstoffen Hemicellulasen, Pentosanasen, Cellulasen, Glucanasen, Alpha-Galactosidasen, Pektinasen, Xylanasen, Phospholipasen, Arabinasen, Zellobiasen oder eine Mischung derselben zugegeben werden. Dabei ist es vorteilhaft, wenn der TS-Gehalt in der Slurry für die Einwirkung der Enzyme ≧ 25 % mas, vorzugsweise ca. 40 % mas beträgt.

Versuche haben ergeben, daß ausschließlich die kombinierte Anwendung von proteinabbauenden Enzymen wie Proteasen und Peptidasen sowie von Hemicellulasen, Pentosanasen, Cellulasen, Glucanasen, Alpha-Galactosidasen, Pektinasen, Xylanasen, Phospholipasen, Arabinasen und Cellobiasen bei gleichzeitigem Eintrag bestimmter moderater Scherkräfte in das Medium eine derartige Abtrennung der Proteinmatrix von der Stärke herbeiführt, so daß eine anschließende Trennung mit hohen Ausbeuten an Protein und Stärke ermöglicht wird. Gleichzeitig ist durch Wahl der hydrolysierenden und modifizierenden Enzyme eine gezielte Modifizierung des Weizenproteins möglich.

Weitere Vorteile dieses Verfahrens sind darin zu sehen, daß im Vergleich zum bekannten Verfahren die Stärkefraktion und die Proteinfraktion einfacher zu trennen sind, und daß das erzeugte Proteinhydrolysat nahezu alle Peptide und Aminosäuren sowohl aus dem glutenbildenden Protein als auch aus dem nichtglutenbildenden Protein des Weizens enthält. Ferner ist für die Herstellung des Proteinhydrolysates gemaß dieser Erfindung der Einsatz eines qualitativ schlechteren Weizens oder Weizenmehles mit einem hohen Anteil an nichtglutenbildendem Protein möglich, da eine Abtrennung des Proteins als Vitalkleber, der ausschließlich nur glutenbildendes Protein enthält, nicht erforderlich ist.

Der dieser Erfindung zugrundeliegende Prozeß ist einfach in seiner Durchführung und führt zu einem Produkt, das sich preislich an dem modifizierten Weizenvitalkleber orientiert. Die gesamten Probleme der Abtrennung von Vitalkleber im konventionellen Prozeß und bei der Verwendung von getrocknetem Vitalkleber für die anschließende Modifizierung, können mit dem erfindungsgemäßen Verfahren umgangen werden. Die Qualität der Zusammensetzung der Aminosäuren in dem Proteinhydrolysat gemäß dieser Erfindung ist höherwertig als beim konventionellen Prozeß, da hier der Anteil des nichtglutenbildenden Proteins ebenfalls enthalten ist. Das Endprodukt weist einen im Vergleich höheren Lysin- und Isoleucingehalt auf.

In der nachfolgenden Zusammenstellung der wichtigsten Aminosäuren werden typische Werte angegeben jeweils für Weizenmehl aus Brotweizen mit 11,5 % mas Protein (auf TS, Berechnungsbasis ist Stickstoff x 5,7), für Weizenvitalkleber aus Brotweizen mit 78 % mas Protein (auf TS, Berechnungsbasis ist Stickstoff x 5,7) und für das Proteinhydrolysat mit 40,6 mas Protein (auf TS, Berechnungsbasis ist Stickstoff x 5,7) aus dem zuvor gesagten Weizenmehl:

| | Weizenmehl (g/100 g Produkt) | Weizenvitalkleber (g/100 g Produkt) | Proteinhydrolysat (g/100 g Produkt) |
|---|---|---|---|
| Isoleucin | 0,46 | 3,0 | 1,78 |
| Leucin | 0,82 | 5,7 | 3,10 |
| Lysin | 0,24 | 1,4 | 1,00 |
| Valine | 0,49 | 3,6 | 1,94 |
| Arginin | 0,43 | 3,2 | 1,8 |
| Glutaminsäure | 3,17 | 31,7 | 12,1 |

Es wird deutlich, daß durch die im Proteinhydrolysat verbleibenden Aminosäuren des nichtglutenbildenden Proteins des Weizens im Vergleich zum Weizengluten und folgerichtig dem daraus hergestellten modifizierten Weizengluten nach dem konventionellen Verfahren höhere Konzentrationen von die Qualität bestimmenden Aminosäuren erzielt werden.

Ein weiterer Vorteil des Proteinhydrolysats nach diesem Verfahren ist seine Säurestabilität, das heißt die Dispersionsstabilität des Proteins bei niedrigen pH-Werten von ca. 4,5 und 10 % mas TS-Gehalt in wässriger Lösung. Dieses ist um so mehr erstaunlich, als der Proteingehalt im Proteinhydrolysat im Vergleich zum konventionell hergestellten modifizierten Weizenkleber mit ca. 40 - 45 % mas Protein auf Gesamt-TS relativ niedrig ist. Diese Säurestabilität ist vorteilhaft bei der Anwendung des Proteinhydrolysats für die Herstellung von Futtermittel für die Jungtieraufzucht.

Dieser relativ niedrige Proteingehalt kann durch Zugabe von getrocknetem Weizenkleber - hergestellt nach dem bekannten Verfahren - oder eines anderen Proteins zu dem Proteinhydrolysat, vorzugsweise zu dem Konzentrat nach der Eindampfung, erhöht werden. Voraussetzung hierfür ist, daß entsprechend der zuvor genannten Maßnahmen auch eine Modifizierung dieses Vitalklebers oder eines anderen Proteins durchgeführt wird. Durch Zugabe von Starprozyme 700 der Firma Biocatalysts Ltd., Main Avenue, Treforest Industrial Estate, Pontypridd, Mid Glamorgan, CF37 5UT, Great Britain wird eine Hydrolysierung des Vitalklebers oder des anderen Proteins vorgenommen. Hierdurch erhöht sich außerdem der Eingangs-TS-Gehalt zur abschließenden Trocknung, wodurch eine Verringerung der spezifischen Trocknungskosten erreicht wird.

Eine zur Durchführung des erfindungsgemäßen Verfahrens geeignete Vorrichtung ist erfindungsgemäß dadurch gekennzeichnet, daß für das Eintragen der Scherkräfte in die Slurry eine Pumpe, zum Beispiel eine Zentrifugalpumpe mit eingebautem Inline-Sieb, eine Mikrokavikationsaufschlußmaschine oder eine Kolloidmühle vorgesehen ist.

Dabei ist es vorteilhaft, wenn der Pumpe eine Verdrängerpumpe vor- und ein statisches Sieb nachgeschaltet sind.

Eine Vorrichtung zur Durchführung des modifizierten Verfahrens gemäß der Erfindung ist vorzugsweise dadurch gekennzeichnet, daß für die der Protein-Hydrolysierung nachgeschaltete Zentrifugation eine Dekanterzentrifuge vorgesehen ist, deren Unterlauf einer zumindest fünf Trennstufen aufweisenden Hydrozyklonanlage zugeführt wird, der für ihren Oberlauf als kombinierte Fest-/Flüssigtrennstufe ein Separator und diesem für seinen Unterlauf ein Dekanter nachgeschaltet sind. Dabei ist es vorteilhaft, wenn zur Trocknung des eingedampften Proteinhydrolysats ein Sprüh- oder Stromtrockner oder eine Mahl-Trocknungsanlage vorgesehen ist.

Das erfindungsgemäße Proteinhydrolysat ist dadurch gekennzeichnet, daß das getrocknete Proteinhydrolysat außer dem Protein weitere Inhaltsstoffe des Weizens oder Weizenmehls wie teilhydrolysierte Pentosane und Glucane, Stärke, Mineralstoffe und niedermolekularen Zucker enthält.

Als Beispiel für die Durchführung des Verfahrens gemäß der Erfindung wird in einen Reaktor - ausgestattet mit einem Rührer, Temperaturregelung und pH-Messung - eine Menge von 3 m³ Wasser mit einer Temperatur von 40 °C eingefüllt. Das Umwälzsystem, bestehend aus einer Verdrängerpumpe mit nachgeschalteter Zentrifugalpumpe mit eingebautem Inline-Sieb und einem statischen Sieb, wird gestartet, bevor ein Weizenmehl mit 10,5 % Protein (berechnet als Stickstoff x Faktor 5,7) entsprechend zudosiert wird.

Als Zentrifugalpumpe wird eine handelsübliche, einstufige Zentrifugalpumpe mit verkleinertem Laufrad, als Inline-Sieb ein Siebeinsatz mit Öffnungen < 1 cm und als statisches Sieb ein umschaltbarer Doppelfilter (z. B. Fabrikat Schünemann Typ F) mit Maschenweiten < 1 mm eingesetzt.

Insgesamt werden zu Beginn 200 kg des Mehles in das Wasser eingemisch, bevor die Enzymmischung Starprozyme 600 der vorstehend genannten Firma zugegeben wird in einer Konzentration von ≦ 1 % mas. Der pH-Wert wird eingestellt mittels Zitronensäure auf einen Wert von 5,5. Anschließend werden weitere 2.160 kg des Weizenmehles eingemischt. Die Reaktion, das heißt die Trennung von Eiweißmatrix und Stärkekörnern, wird mittels eines Mikroskops verfolgt. Nach ca. 1 Stunde Reaktionszeit ist die Reaktion beendet und die Slurry wird zu einem 2-Phasen-Dekanter gepumpt, in welchem eine Trennung in Unter- und Oberlauf erfolgt. Der Unterlauf hat eine Gesamt-TS von 55 % mas bei einem Proteingehalt von 3,0 % mas (Berechnungsbasis ist Stickstoff x Faktor 5,7). Der Oberlauf weist eine Gesamt-TS von 17,2 % mas sowie eine Proteinkonzentration von 41,6 % mas (Berechnungsbasis ist Stickstoff x Faktor 6,25) auf. Der Oberlauf des Dekanters wird in einer Eindampfanlage nach Einstellung des pH-Wertes mit Zitronensäure von pH 4,4 auf eine Endkonzentration von 40,5 % mas TS konzentriert. Das Proteinhydrolysat wird anschließend in einem Sprühtrockner getrocknet. Das erhaltene Endprodukt hat einen pH-Wert von 4,4, eine Proteinkonzentration von 41 % mas (Berechnungsbasis ist Stickstoff x 6,25), eine Gesamttrockensubstanz von 93,8 % mas, einen Hydrolysegrad des Proteins von 8,5 (entsprechend der Bestimmungsmethode für den Hydrolysegrad [DH-Wert] aus J. Adler Nissen: Determination of Degree of Hydrolysis of Food Protein Hydrolysates by TNBS und J. Agric Food Chem., Vol. 27, No. 6, 1979, p. 1256 - 1262) und eine Säurestabilität von mindestens zwölf Stunden.

Die Säurestabilität wird bestimmt durch die qualitative Beurteilung des Absetzverhaltens von 10 %igen Lösungen (% mas) bei ph-Wert 4,5 nach zwölf Stunden.

Die erfindungsgemäße Stärkemilch ist dadurch gekennzeichnet, daß die unraffinierte Stärkemilch eine Stärkekorngrößenverteilung annähernd der des eingesetzten Weizenmehles aufweist und außer der Stärke weitere Inhaltsstoffe des Weizenmehles wie teilhydrolysierte Pentosane, Glucane, Proteine, Mineralstoffe und niedermolekulare Zucker enthält.

Für die Erzielung einer bestmöglichen Stärkeausbeute sind die folgenden Vorkehrungen notwendig:
- der Einsatz von Proteasen und/oder Peptidasen;
- Einstellung der physikalischen Parameter des Prozesses (Temperatur, pH-Konzentration) in Übereinstimmung mit den Wirkungsoptima der Enzyme;
- Einsatz von Enzymen, die eine maximale Reduzierung der Viskosität, also einen Abbau der unlöslichen Pentosane in der Slurry bewirken;
- die Eintragung von Scherkräften in die Slurry kann im Vergleich zu den bekannten Prozessen moderat gehalten werden;
- die verwendete Weizenmehlqualität muß nicht die gleichen hohen Anforderungen erfüllen wie bei den konventionellen Prozessen.

Für die Durchführung eines modifizierten Verfahrens gemäß der Erfindung wird dem nach dem Reaktionsbereich eingesetzten 2-Phasen-Dekanter ein Gesamtmassenstrom von 5.360 kg zugeführt. Der Oberlauf beträgt 2.492 kg, der Unterlauf 2.868 kg. Dieser Unterlauf enthält eine Gesamt-TS von 55 % mas bei einem Proteingehalt von 3,5 % mas (Berechnungsbasis Stickstoff x 5,7). Bezogen auf die Trockensubstanz beträgt der Zufluß zum Dekanter 2.006 kg, der Oberlauf 428,6 kg und der Unterlauf 1.577,4 kg. Die entsprechenden Proteinanteile betragen im Zulauf 210,6 kg, im Oberlauf 162,6 kg und im Unterlauf 48 kg.

Zu den bekannten Prozessen bestehen bemerkenswerte Unterschiede:
- Hier wird in dem 3-Phasen-Dekanter-Prozeß eine hohe Scherkraft in die Slurry eingetragen durch z. B. eine Hochdruckpumpe oder einen Homogenisator. In dem Hydrozyklonprozeß wird die hohe Scherkraft durch die Anwendung mehrerer Hydrozyklonstufen eingetragen. In dem dieser Erfindung zugrundeliegendem Verfahren werden hingegen lediglich moderate Scherkräfte in der Slurry angewendet durch Umpumpen der Slurry mittels einer Zentrifugalpumpe mit eingebautem Inline-Sieb.
- In dem 3-Phasen-Dekanter-Prozeß beträgt der TS-Gehalt des Zulaufes für den 3-Phasen-Dekanter maximal ca. 32 % mas, in dem Hydrozyklon-Prozeß ist der TS-Gehalt im Zulauf auf ca. 16 % mas begrenzt. In dem Verfahren gemäß dieser Erfindung beträgt der TS-Gehalt im Zulauf zum 2-Phasen-Dekanter 37,5 %. Dieses wird ermöglicht durch die Erniedrigung der Viskosität in der Slurry und durch den mittels der Enzyme vorgenommenen Proteinabbau.
- Aufgrund der höheren Löslichkeit des Proteins durch die enzymatische Behandlung liegt der Proteingehalt im Unterlauf des 2-Phasen-Dekanters mit 3 % mas auf TS höher als im Unterlauf des Dekanters im 3-Phasen-Dekanter-Prozeß. Es ist jedoch sehr leicht möglich, diese in der anschließenden mit Hydrozyklonen vorgenommenen Raffination weitestgehend zu entfernen, da es sich hierbei fast ausschließlich um lösliches Protein handelt.
- Da der Unterlauf des 2-Phasen-Dekanters gemäß des dieser Erfindung zugrundeliegenden Verfahrens mit 78,6 % mas bezogen auf den Zulauf zu diesem Dekanter im Vergleich mit dem 3-Phasen-Dekanter-Prozeß, in welchem dieser Massenanteil lediglich 60 % beträgt, sehr hoch ist, ist hieraus schon ersichtlich, daß fast die gesamte Stärke in dem Unterlauf enthalten ist und zur Raffination gelangen kann. Die Ausbeute an qualitativ hochwertiger Stärke beträgt ca. 78 % im Vergleich zu ca. 60 % bei den konventionellen Prozessen.

Die eingangs formulierte weitere Aufgabe wird erfindungsgemäß gelöst durch die Verfahrensschritte gemäß Anspruch 24.

Nach einer Ausführungsform des Verfahrens werden Endprodukte gemeinsam mit Zwischenprodukten eingesetzt. Im Fall, daß Vitalkleber als Endprodukt Verwendung findet, ist es notwendig, das Einsatzmaterial zu lösen bzw. zu de-agglomerieren. Dieses kann dadurch geschehen, daß das Gesamteinsatzprodukt mit der Enzymmischung Starprozyme 710 der Firma Biocatalysts Ltd., Main Avenue, Treforest Industrial Estate, Pontypridd, Mid Glamorgan, CF37 5UT, Großbritannien, in einer Konzentration ≦ 1 % mas bezogen auf die Trockensubstanz des Gesamteinsatzmediums versetzt wird und diese Mischung auf der Saugseite einer Verdrängerpumpe zugeführt wird. Die Druckseite der Verdrängerpumpe ist verbunden mit einem Satz doppelmanteliger statischer Mischer der Fa. StaPro Mix BV, und wird durch diese umgepumpt. Im Anschluß an die statischen Mischer wird durch Wahl der Größe der Zuführungsleitung in den Reaktionsbereich eine bestimmte Aufenthaltszeit für diese Mischung vorgegeben, so daß eine weitestgehende De-agglomeration erreicht wird. Die Beheizung der Doppelmäntel der statischen Mischer zur Einstellung einer Reaktionstemperatur von vorzugsweise ca. 45 °C wid mit Warmwasser von 80 °C durchgeführt.

Eine Inaktivierung der eingesetzten Enzyme kann mit einem Verfahren gemäß Anspruch 40 erfolgen.

In der Zeichnung sind einige als Beispiele dienende Ausführungsformen der Erfindung in Form von Blockdiagrammen dargestellt. Es zeigen:
- **Figur 1**: ein Verfahren zur Gewinnung von Weizenproteinhydrolysat;
- **Figur 2**: ein gegenüber Figur 1 nur leicht modifiziertes Herstellungsverfahren für ein Proteinkonzentrat;
- **Figur 3**: einen Weizenstärkeprozeß mit getrennter enzymatischer Behandlung von B-Stärke und C-Stärke, Vitalkleber und Weizenproteinhydrolysat-Herstellung (WPH) sowie möglicher Konzentrationsanpassung des WPH's durch Verwendung von Vitalkleber und/oder anderen Proteinquellen und/oder Weizenmehl;
- **Figur 4**: einen Weizenstärkeprozeß mit enzymatischer B-Stärke- und C-Stärke-Behandlung, Vitalkleber und Weizenproteinhydrolysat-Herstellung (WPH) sowie möglicher Konzentrationsanpassung des WPH's durch Verwendung von Vitalkleber und/oder anderen Proteinquellen und/oder Weizenmehl;
- **Figur 5**: einen Weizenstärkeprozeß mit enzymatischer Behandlung der Protein- und B-Stärke sowie C-Stärke enthaltenen Phasen nach der A-Stärke-Abtrennung sowie Konzentrationsanpassung des WPH's durch Verwendung von Vitalklebern und/oder anderen Proteinquellen und/oder Weizenmehl; und
- **Figur 6**: einen Weizenstärkeprozeß mit enzymatischer Behandlung der Protein- und B-Stärke sowie C-Stärke enthaltenen Phasen nach der A-Stärke-Abtrennung.

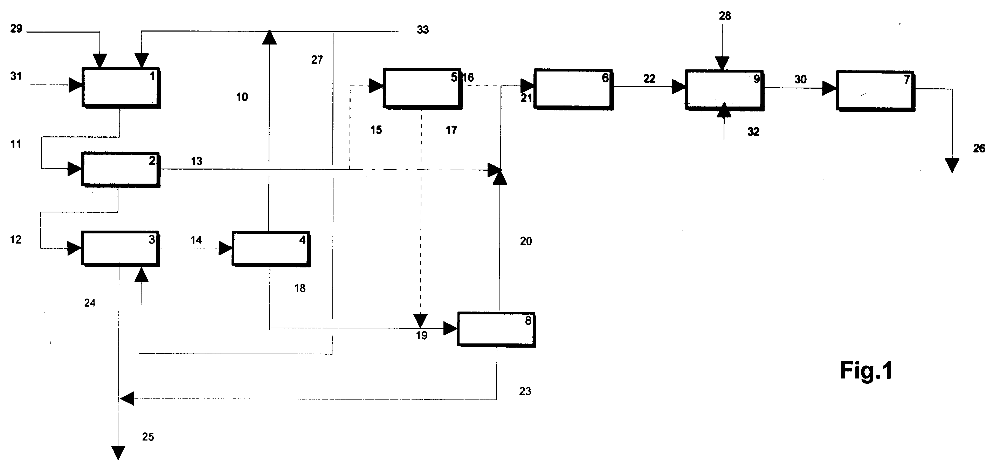

Gemäß Figur 1 wird in einem Reaktionsbereich 1 eine Mischung hergestellt aus Wasser 33 und Weizenmehl 29 bei Einhaltung eines TS-Gehaltes von ≧ 25 % mas, vorzugsweise 40 % mas, einer Temperatur von < 45 °C und einem pH-Wert von > 4,0 und < 7,0, vorzugsweise > 5,0 und < 6,0. Zugegeben wird dann eine Enzymmischung 31, die Proteasen und Peptidasen zur Hydrolysierung des glutenbildenden und nichtglutenbildenden Proteins und zusätzlich Hemicellulasen, Pentosanasen, Cellulasen, Glucanasen, Alpha-Galactosida-sen, Pektinasen, Xylanasen, Phospholipasen, Arabinasen, Zellobiasen zum Abbau von nichtprotein-und nichtstärkehaltigen Inhaltsstoffen wie z. B. Hemicellulose, Pentosane und Glukane aufweist. Die Enzyme werden als definierte Enzymmischung 31 mit einer Gesamtkonzentration < 1 % mas auf TS zugegeben.

Der Reaktionsbereich 1 wird durch einen Reaktionsbehälter gebildet, der mit einem Rührelement, einer Temperaturregelung und einem Umwälzsystem, bestehend aus einer Verdrängerpumpe mit nachgeschalteter Zentrifugalpumpe mit eingebauten Inline-Sieb und einem statischen Sieb, ausgestattet ist.

Die Reaktionszeit ist abhängig von den bereits oben genannten Parametern Temperatur, pH-Wert, Enzymkonzentration in der Slurry, Mischungsverhältnisse der Enzyme und deren Auswahl, Qualität des Rohstoffes sowie von dem eingesetzten Umwälzsystem zur Eintragung von Scherkräften. Der Verlauf der Reaktion kann durch mikroskopische Kontrolle beobachtet werden. Die erfindungsgemäße Durchführung des Verfahrens führt dazu, daß das Protein derart gelöst und/oder dispergiert wird, daß die Proteinmatrix aufgelöst und die Stärkekörner freigesetzt werden. Während der Reaktion findet ein signifikanter Abfall der Viskosität in der Slurry statt, wodurch die nachfolgenden Trennschritte erheblich vereinfacht werden. Nach Abschluß der Reaktion wird das Reaktionsgemisch 11 in einem Zentrifugalfeld 2, z. B. mittels einer Dekanterzentrifuge ohne vorhergehende Verdünnung getrennt. Als Dekanterzentrifuge wird ein 2-Phasen-Dekanter eingesetzt, der es erlaubt, einen hohen TS-Gehalt im Unterlauf, ein Minimum an Stärkepartikeln im Oberlauf und einen maximalen Durchsatz zu erreichen. Die Dekanterzentrifuge sollte eine Einstellmöglichkeit für die Differenzdrehzahl zwischen Schnecke und Zentrifugenkörper aufweisen.

Der Oberlauf 13 der Dekanterzentrifuge 2 wird anschließend in einer Eindampfanlage 6 konzentriert. Alternativ ist es möglich, den Proteingehalt des Dekanteroberlaufes 13, 15 durch einen nachgeschalteten Separator 5 zuvor weiter aufzukonzentrieren.

Der pH-Wert des Oberlaufes 13 sollte vor der Eindampfung mittels einer organischen Säure wie z. B. Zitronensaure, Milchsäure oder durch Anwendung einer Fermentation mit säureproduzierenden Mikroorganismen auf einem Wert von ca. 4,0 eingestellt werden.

Der Unterlauf 12 des Dekanters 2 kann weiter raffiniert werden in einer Gegenstrom-Hydrozyklon-Raffination 3 zur Reinigung der enthaltenen Stärke bei Einsatz von Wasser 27. Der Unterlauf 24 der letzten Hydrozyklonstufe enthält die Stärke und ist nach einer Faserabtrennung geeignet für eine weitere Verarbeitung zu z. B. Stärkederivaten. Der Oberflauf 14 des ersten Hydrozyklons kann durch eine kombinierte Anwendung von Separator 4 und Dekanter 8 aufkonzentriert werden. Der Oberlauf 10 dieses Separators 4 kann zum Anmischen für das Mehl und somit zur Reduzierung des benötigten Frischwassers verwendet werden. Der Oberlauf 20 dieses Dekanters 8 kann zusammen mit dem Oberlauf 16 des Separators 5 oder aber alternativ mit dem Oberlauf 13 des Dekanters 2 als Zulauf 21 zu der Eindampfanlage 6 geführt werden. Der Unterlauf 17 des Separators 5 kann zusammengeführt werden mit dem Unterlauf 18 des Separators 4 und bildet dann den Zulauf 19 zum Dekanter 8. Der Unterlauf 23 des Dekanters 8 wird mit dem Unterlauf 24 der Hydrozyklone (3) zu einem Stärkemilchprodukt 25 vereinigt.

Das Konzentrat 22 aus der Eindampfanlage 6 kann in dem Behälter 9 mit Vitalkleber 28 angereichert und anschließend durch Zugabe von Enzymen 32 behandelt sowie anschließend getrocknet werden in einem Sprühtrockner 7 oder in einem Stromtrockner, der mit einem entsprechendem Rückmischsystem ausgestattet ist. Als Endprodukt 26 resultiert das getrocknete Proteinkonzentrat.

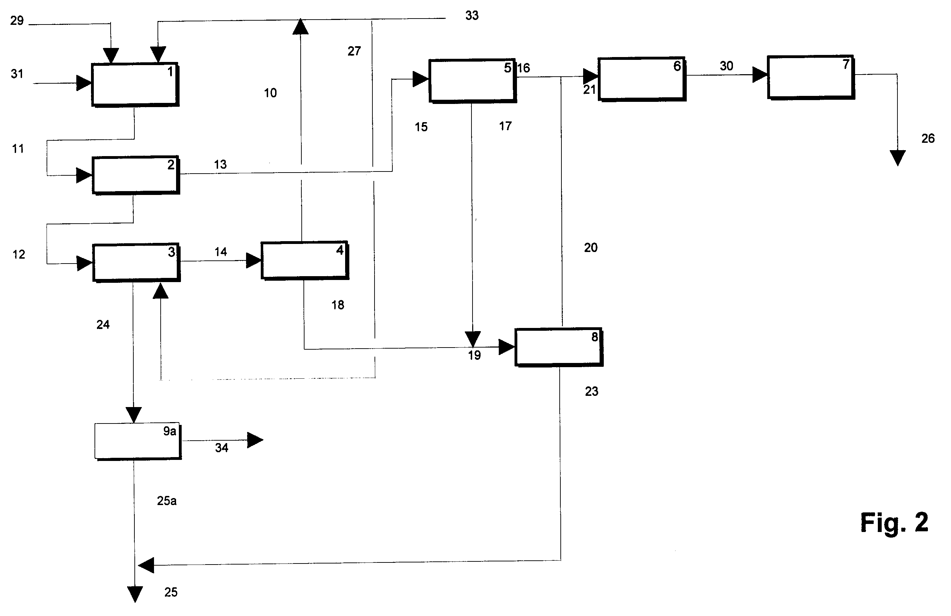

Figur 2 zeigt in einem Blockdiagramm ein gegenüber Figur 1 nur leicht modifiziertes Verfahren gemäß der Erfindung:
In einem Reaktionsbereich 1 wird eine Mischung hergestellt aus Wasser 33 und Weizenmehl 29 bei Einhaltung eines TS-Gehaltes von ≧ 25 % mas, vorzugsweise 40 % mas, einer Temperatur von < 60 °C und einem pH-Wert von > 4,0 und < 7,0, vorzugsweise > 5,0 und < 6,0. Zugegeben wird dann eine Enzymmischung 31, die Proteasen und Peptidasen zur Hydrolysierung des glutenbildenden und nichtglutenbildenden Proteins und zusätzlich Hemicellulasen, Pentosanasen, Cellulasen, Glucanasen, Alpha-Galactosidasen, Pektinasen, Xylanasen, Phospholipasen, Arabinasen, Cellobiasen zum Abbau von nichtprotein- und nichtstärkehaltigen Inhaltsstoffen wie z. B. Hemicellulose, Pentosane und Glucane aufweist. Die Enzyme werden als definierte Enzymmischung 31 zugegeben.

Der Reaktionsbereich 1 wird durch einen Reaktionsbehälter gebildet, der mit einem Rührelement, einer Temperaturregelung und einem Umwälzsystem, bestehend aus einer Verdrängerpumpe mit nachgeschalteter Zentrifugalpumpe mit eingebautem Inline-Sieb und einem statischen Sieb, ausgestattet ist.

Die Reaktionszeit ist abhängig von den bereits oben genannten Parametern Temperatur, pH-Wert, Enzymkonzentration in der Slurry, Mischungsverhältnisse der Enzyme und deren Auswahl, Qualität des Rohstoffes sowie von dem eingesetzten Umwälzsystem zur Eintragung von Scherkräften. Der Verlauf der Reaktion kann durch mikroskopische Kontrolle beobachtet werden. Die erfindungsgemäße Durchführung des Verfahrens führt dazu, daß das Protein derart gelöst und/oder dispergiert wird, daß die Proteinmatrix aufgelöst und die Stärkekörner freigesetzt werden. Während der Reaktion findet ein signifikanter Abfall der Viskosität in der Slurry statt, wodurch die nachfolgenden Trennschritte erheblich vereinfacht werden. Nach Abschluß der Reaktion wird das Reaktionsgemisch 11 in einem Zentrifugalfeld 2, z. B. mittels einer Dekanterzentrifuge ohne vorhergehende Verdünnung getrennt. Als Dekanterzentrifuge wird ein 2-Phasen-Dekanter eingesetzt, der es erlaubt, einen hohen TS-Gehalt im Unterlauf 12, ein Minimum an Stärkepartikeln im Oberlauf 13 und einen maximalen Durchsatz zu erreichen. Die Dekanterzentrifuge sollte eine Einstellmöglichkeit für die Differenzdrehzahl zwischen Schnecke und Zentrifugenkörper aufweisen.

Der Oberlauf 13 der Dekanterzentrifuge 2 wird anschließend in einem Separator 5 weiter aufkonzentriert und anschließend in einer Eindampfanlage 6 konzentriert. Der pH-Wert des Oberlaufes 16 des Separators 5 sollte vor der Eindampfung 6 mittels einer organischen Säure wie z. B. Zitronensäure, Milchsäure oder durch Anwendung einer Fermentation mit säureproduzierenden Mikroorganismen auf einem Wert von ca. 4,0 eingestellt werden.

Der Unterlauf 12 des Dekanters 2 kann weiter raffiniert werden in einer Gegenstrom-Hydrozyklon-Raffination 3 zur Reinigung der enthaltenen Stärke bei Einsatz von Wasser 27. Der Unterlauf 24 der letzten Hydrozyklonstufe enthält die Stärke und ist nach Ausscheidung der Fasern 34 in einer Faserabtrennung 9 als gereinigter Stärkestrom 25a geeignet für eine weitere Verarbeitung zu z. B. Stärkederivaten. Der Oberlauf 14 der ersten Stufe der Hydrozyklon-Raffination 3 kann durch eine kombinierte Anwendung von Separator 4 und Dekanter 8 aufkonzentriert werden. Der Oberlauf 10 dieses Separators 4 kann zum Anmischen für das Mehl und somit zur Reduzierung des benötigten Frischwassers verwendet werden. Der Oberlauf 20 dieses Dekanters 8 kann zusammen mit dem Oberlauf 16 des Separators 5 als Zulauf 21 zu der Eindampfanlage 6 geführt werden. Der Unterlauf 17 des Separators 5 wird zusammengeführt mit dem Unterlauf 18 des Separators 4 und bildet dann den Zulauf 19 zum Dekanter 8. Dessen Unterlauf 23 wird anschließend mit dem gereinigten Stärkestrom 25a zu einem Stärkemilchprodukt 25 vereinigt.

Das konzentrierte Proteinhydrolysat 30 aus der Eindampfanlage 6 kann anschließend getrocknet werden in einem Sprühtrockner 7 oder in einem Stromtrockner, der mit einem entsprechendem Rückmischsystem ausgestattet ist. Als Endprodukt 26 resultiert das getrocknete Proteinkonzentrat.

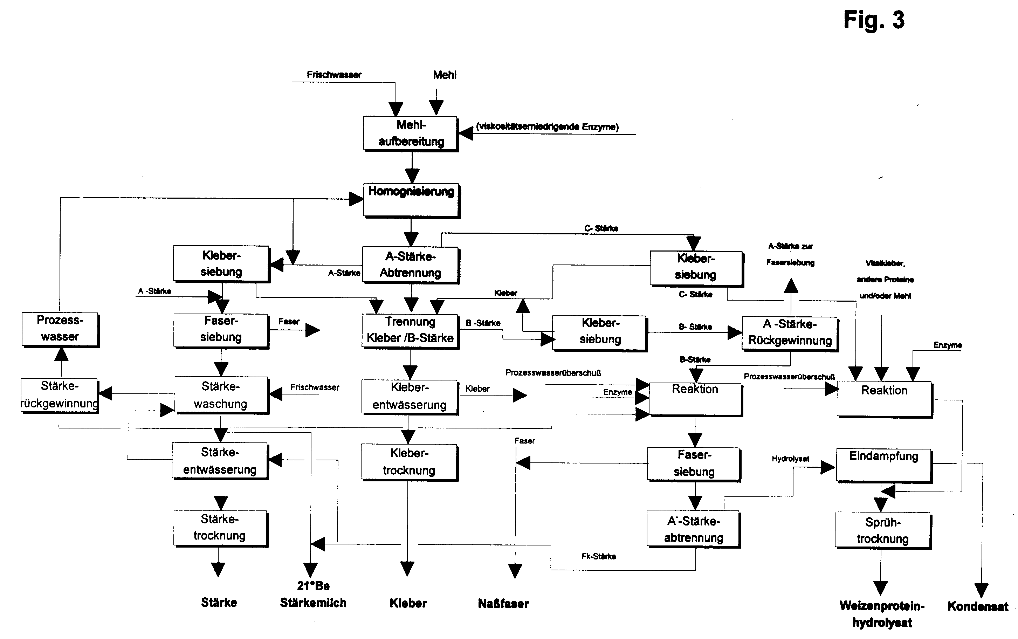

Figur 3 beschreibt die enzymatische Behandlung von C-Stärke unter Einsatz von Prozeßwasser, wobei im Anschluß daran eine Reinigung der Stärkefraktion durch Absiebung der Faserstoffe und Feinkornstärkeabtrennung vorgenommen werden. Diese Behandlung geschieht in Verbindung mit der Gewinnung von A-Stärke nach dem üblichen konventionellen Verfahren zur Weizenstärkeherstellung, wobei Vitalkleber und A-Stärke in einem 3-Phasen-Dekanter-Prozeß gewonnen werden. Als zusätzliche Produkte nach der Erfindung werden A⁻-Stärke und Weizenproteinhydrolysat gewonnen. Die enzymatische Behandlung von B-Stärke und C-Stärke wird getrennt vorgenommen, wobei der enzymatischen Behandlung der C-Stärke Vitalkleber als Endprodukt der Weizenstärkeherstellung, Weizenmehl als Ausgangsstoff der Weizenstärkeherstellung oder auch andere Proteine zugegeben werden können. Hierdurch ist es möglich, die Einstellung der Proteinkonzentration in dem gemeinsam nach einer Eindampfung des Hydrolysates aus der B-Stärke-Behandlung einer Sprühtrocknung zugeführten Gesamthydrolysates vorzunehmen. Die Kapazität der enzymatischen Behandlungsanlage wird sinnvollerweise der Gesamtverarbeitungskapazität der Weizenstärkeanlage angepaßt, so daß die dort anfallende Zwischenproduktmenge an B-Stärke und C-Stärke sowie das überschüssige Prozeßwasser verarbeitet werden können.

Es ist offensichtlich, daß es bei dieser Ausführungsform des Verfahrens nach der Erfindung keine technischen und technologischen Abhängigkeiten zwischen dem konventionellen Teil der Weizenstärkeherstellung, d. h. der A-Stärke-Abtrennung mittels 3-Phasen-Dekanter und deren anschließenden Reinigung sowie der Behandlung der B-Stärke, C-Stärke und Prozeßwassernebenprodukte gibt. Dieses wird dadurch erreicht, daß sichergestellt wird, daß Enzym enthaltenes Prozeßwasser nicht zu den Prozeßschritten des konventionellen Verfahrens zurückgeführt wird.

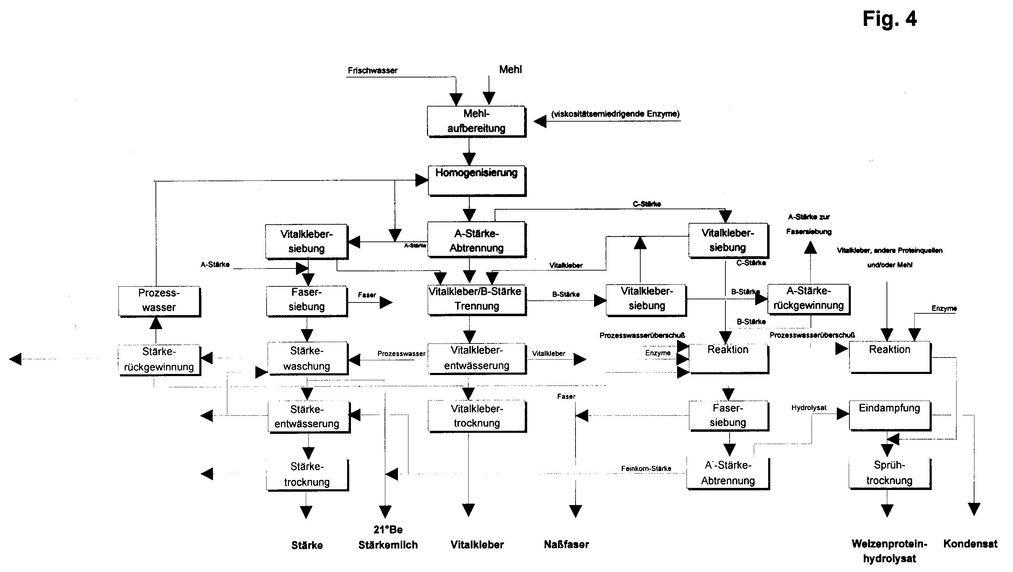

Figur 4 zeigt die enzymatische Behandlung der B-Stärke und C-Stärke-Endprodukte in einer gemeinsamen enzymatischen Behandlungsstufe. Die A⁻-Stärke wird ebenfalls nach der enzymatischen Reaktion aus dem Reaktionsgemisch abgetrennt und zurückgewonnen. Eine vorhergehende Abtrennung der Faserbestandteile der B-Stärke und C-Stärke-Endprodukte ist vorgesehen. Als zusätzliche Produkte resultieren aus dieser Verfahrensvariante ebenfalls A⁻-Stärke und ein Weizenproteinhydrolysat. Zusätzlich können in einer getrennten aber gleichzeitig hierzu ablaufenden enzymatischen Reaktion Vitalkleber als Endprodukt der Weizenstärkeherstellung, Weizenmehl als Ausgangsstoff der Weizenstärkeherstellung oder andere Proteine behandelt werden und anschließend gemeinsam mit dem konzentrierten Reaktionsgemisch aus der B- und C-Stärke-Behandlung einer Sprühtrocknung zugeführt werden. Hierdurch kann der Proteingehalt in dem Weizenproteinhydrolysat eingestellt werden.

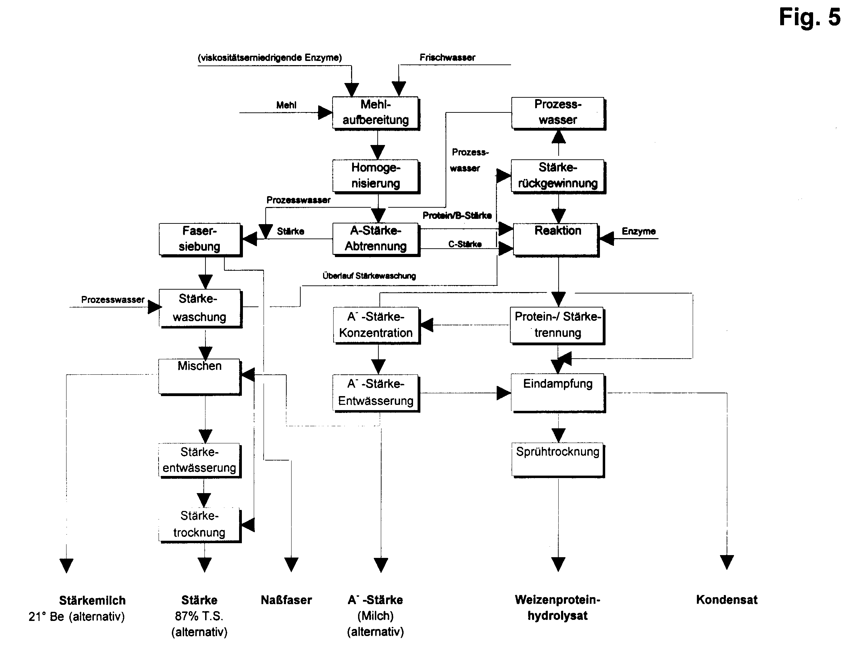

Figur 5 beschreibt die gemeinsame enzymatische Behandlung der B-Stärke sowie der C-Stärke mit der Vitalglutenfraktion, die bei der A-Stärke-Abtrennung mittels 3-Phasen-Dekanter-Prozeß neben der A-Stärke als weitere Phasen gewonnen werden. Im Anschluß an die enzymatische Reaktion wird eine Protein/Stärketrennung ebenfalls mittels Dekanter vorgenommen und die so abgetrennte A⁻-Stärke konzentriert und als Stärkemilch oder alternativ gemeinsam mit der A-Stärke gewonnen. Überschüssiges Prozeßwasser, welches bei der A⁻-Stärke-Abtrennung anfällt, wird gemeinsam mit dem Reaktionsgemisch nach der Stärkeabtrennung einer Eindampfung zugeführt und anschließend sprühgetrocknet.

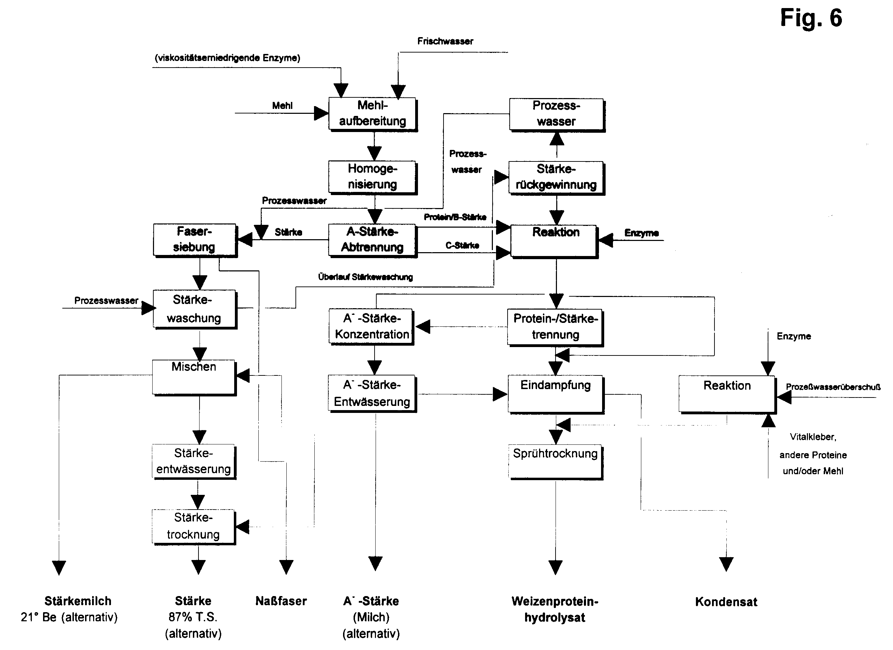

Gemäß Figur 6 werden die B-Stärke sowie die C-Stärke gemeinsam mit der Vitalkleberfraktion sowie dem Überschußprozeßwasser- wie bereits in dem vorhergehenden Beispiel beschrieben - enzymatisch behandelt. Zur Einstellung und Anpassung der gewünschten Proteinkonzentration im hergestellten Weizenproteinhydrolysat wird einhergehend mit der zuvor beschriebenen enzymatischen Behandlung Vitalkleber als Endprodukt, Weizenmehl als Ausgangsprodukt und/oder andere Proteine einer enzymatischen Reaktion unterworfen, wobei das Reaktionsgemisch gemeinsam mit dem in einer Eindampfung aufkonzentrierten Reaktionsgemisch aus der B-Stärke- und C-Stärke-Behandlung resultierenden Konzentrat einer Sprühtrocknung zugeführt wird.

Bei dieser Prozeßführung resultieren als zusätzliche Produkte A⁻-Stärke und Weizenproteinhydrolysat.

Ein weiteres Beispiel für die Durchführung eines Verfahrens nach dieser Erfindung stellt die gemeinsame enzymatische Behandlung der B-Stärke, C-Stärke und des Prozeßwasserüberschusses dar. Im Anschluß an die enzymatische Reaktion wird die A⁻-Stärke zurückgewonnen. Als Produkte werden A⁻-Stärke und Weizenproteinhydrolysat erhalten. Auch hierbei besteht die Möglichkeit durch gleichzeitige getrennte enzymatische Behandlung von Vitalkleber als Endprodukt und/oder Weizenmehl als Ausgangsstoff und/oder anderer Proteinquellen eine Anpassung der Proteinkonzentration im Weizenproteinhydrolysat vorzunehmen. Unterschiedlich zu der im vorhergehenden Beispiel genannten Vorgehensweise wird in diesem Fall der von dem Vitalkleber getrennte B-Stärke-Strom behandelt, so daß eine Vitalklebergewinnung nach Siebung des B-Stärke-Stromes möglich ist.

Wie bereits aus den zuvor genannten Beispielen ersichtlich, gibt es eine Vielzahl von möglichen Kombinationen der Endprodukte, Zwischenprodukte und Ausgangsstoffe bei der Weizenstärkeherstellung, welche durch Anpassung der jeweiligen Prozeßführung einer enzymatischen Behandlung zur Gewinnung von Weizenproteinhydrolysat angewendet werden können. Die genannten Beispiele beziehen sich auf den Einsatz des 3-Phasen-Dekanter-Prozesses für die Abtrennung der A-Stärke. Es ist ebenso möglich, hierfür in Kombination 2-Phasen-Dekanter und/oder Hydrozyklone 2stufig einzusetzen.

Im ersten Schritt eines Ausführungsbeispiels gemäß Figur 3 wird eine enzymatische Behandlung von B-Stärke als Endprodukt und Prozeßwasser vorgenommen. Hierzu werden 2,3 m³ einer B-Stärke mit 14,1 % Trockensubstanz und 2,6 % Protein auf Trockensubstanz mit 0,3 m³ Prozeßwasser mit einer Trockensubstanz von 4,8 % und einem Proteingehalt von 10,2 % auf Trockensubstanz gemischt und in einen Reaktionsbehälter gepumpt, welcher mit einem Rührwerk, einer Pumpe zum Umpumpen der Mischung durch ein Sieb sowie einer pH-Meßeinrichtung und einer Temperaturregeleinrichtung ausgestattet ist. Der pH-Wert wird mit Zitronensäure eingestellt auf pH 5,5 und die Temperatur wird auf 40 °C geregelt. Das Gesamtvolumen des Reaktionsbehälters beträgt 3 m³, die Kapazität der Pumpe 12 m³/h mit einer Förderdruckdifferenz von 6 Bar. Das Sieb ist ausgestattet mit Löchern < 1 cm im Durchmesser. Nachdem das Gemisch in den Reaktionsbehälter überführt ist, wird das Enzymgemisch Starprozyme 720 der Firma Biocatalysts Ltd., Main Avenue, Teforest Industrial Estate, Pontypridd, Mid Glamorgan/CF37 5UT, Großbritannien, zugegeben. Die Reaktionszeit beträgt 30 Minuten. Die eintretende Trennung der Stärkepartikel von der Protein/Stärkematrix wird durch fortlaufende Untersuchungen mittels eines Mikroskops überwacht. Nach weitestgehender De-agglomerierung der Protein/Stärkematrix wird das Reaktionsgemisch über einen Starcosa-Strahlauswascher mit einer Siebbespannung (Siebröße 90 µm) filtriert, wobei die Faserbestandteile abgetrennt werden. Die im Siebdurchtritt erhaltene unreine Stärkesuspension wird anschließend in einem 2-Phasen-Dekanter in eine überwiegend Stärke enthaltene Unterphase und eine überwiegend Protein enthaltene Oberphase getrennt. Die Oberphase wird anschließend in einer Eindampfanlage aufkonzentriert.

Im zweiten Schritt des Ausführungsbeispiels gemäß Figur 3 wird eine C-Stärke als Endprodukt gemeinsam mit einem getrockneten Vitalkleber als Endprodukt und einem Prozeßwasser einer Weizenstärkeherstellung gemeinsam enzymatisch behandelt. Hierzu werden 1,3 m³ der C-Stärke mit 14,7 % Trockensubstanz und einem Proteingehalt von 12,7 % auf Trockensubstanz mit 0,3 m³ Prozeßwasser mit einem Trockensubstanzgehalt von 4,8 % und einem Proteingehalt von 10,2 % auf Trockensubstanz in einen Reaktionsbehälter zugeführt, der dem des ersten Schritts entspricht. Die Temperatur wird ebenfalls auf 40 °C geregelt, und der pH-Wert wird auf pH 5,5 eingestellt. Anschließend wird 1 t getrockneter Vitalkleber mit 96 % Trockensubstanz und 82,3 % Proteingehalt auf Trockensubstanz in den Reaktor zugeführt. Für die enzymatische Behandlung wird eine Enzymmischung Starprozyme 730 der Firma Biocatalysts Ltd., Main Avenue, Treforest Industrial Estate, Pontypridd, Mid Glamorgan, CF37 5UT, Großbritannien, zugegeben. Die anschließend eingehaltene Reaktionszeit für die Enzymeinwirkung beträgt 1 Stunde. Das Reaktionsgemisch wird letztendlich zusammengeführt mit dem Konzentrat nach der Eindampfung wie unter Schritt 1 beschrieben. Diese neue Mischung wird anschließend auf eine Temperatur von 80 °C angewärmt, um die noch enthaltenen aktiven Enzyme zu deaktivieren bei gleichzeitiger Einstellung des pH-Wertes auf pH 4,4. Anschließend wird diese Mischung in einem Sprühtrockner auf 96 % TS getrocknet.

Als Produkte resultieren eine A⁻-Stärke, enthalten im Unterlauf des Dekanters wie unter Schritt 1 beschrieben mit einer Konzentration von 49,2 % Gesamttrockensubstanz sowie ein Weizenproteinhydrolysat mit einem Hydrolysegrad des Proteins von 9,1 (entsprechend der Bestimmungsmethode für den Hydrolysegrad [DH-Wert] aus J. Adler Nissen: Determination of Degree of Hydrolysis of Food Protein Hydrolysates by TNBS un J. Agric Food Chem., Vol. 27, N.6, 1979, p. 1256 - 1262) und einem Proteingehalt (Berechnungsbasis ist Stickstoff x 6,25) von 65,4 % auf Trockensubstanz.

## Patentansprüche

1. Verfahren zur Gewinnung von Weizenstärke und/oder Weizenproteinhydrolysat aus Weizen, geschältem Weizen, Weizenmehl oder proteinangereichertem Weizenmehl, wobei der Weizen oder geschälte Weizen zuerst trocken oder naß vermahlen wird, das Weizenmehl oder proteinangereicherte Weizenmehl ausschließlich naß vermahlen werden kann und danach mit Wasser bei Temperaturen ≦ 50 °C zu einer Slurry verarbeitet wird, die anschließend durch Zentrifugieren getrennt wird in eine Stärke und Fasern enthaltende Fraktion und in eine glutenbildende Proteinfraktion, **gekennzeichnet durch** folgende Verfahrensschritte:
a) vor dem Zentrifugieren werden der Slurry Enzyme zugegeben, um das glutenbildende und nicht glutenbildende Weizenprotein sowie weitere nicht protein- und nicht stärkehaltige Inhaltsstoffe in der Slurry zu einem Weizenproteinhydrolysat zu hydrolysieren;
b) die Slurry wird auf die gewünschte Temperatur sowie auf einen pH-Wert > 4,0 und < 7,0 eingestellt;
c) in die Mischung werden Scherkräfte eingetragen und zwar durch Umpumpen der Mischung durch ein Sieb, wobei den Feststoffanteilen der Mischung unmittelbar vor dem Sieb mittels einer eingesetzten Pumpe eine hohe Beschleunigung erteilt wird.

2. Verfahren nach Anspruch 1, **dadurch gekennzeichnet**,
daß das in den Reaktionsbereich zugegebene, eine Temperatur von < 60 °C, vorzugsweise 40 °C aufweisende Wasser gerührt und umgepumpt wird;
daß dem umgepumpten Wasser eine erste Teilmenge Weizenmehl zudosiert wird;
daß in die so gebildete Slurry Scherkräfte eingetragen werden;
daß nach der Enzymzugabe die Einstellung des pH-Wertes auf > 5,0 und < 6,0 erfolgt; und
daß unter Aufrechterhaltung der Scherkrafteintragung die Zudosierung der zweiten Weizenmehl-Teilmenge erfolgt.

3. Verfahren nach Anspruch 1 oder 2, **dadurch gekennzeichnet**, daß für die Hydrolysierung und/oder Modifizierung des glutenbildenden und nichtglutenbildenden Proteins Proteasen und Peptidasen, für den Abbau von nicht protein- und nicht stärkehaltigen Inhaltsstoffen Hemicellulasen, Pentosanasen, Cellulasen, Glucanasen, Alpha-Galactosidasen, Pektinasen, Xylanasen, Phospholipasen, Arabinasen, Zellobiasen oder eine Mischung derselben zugegeben werden.

4. Verfahren nach einem der vorhergehenden Ansprüche, **dadurch gekennzeichnet**, daß der TS-Gehalt in der Slurry für die Einwirkung der Enzyme ≧ 25 % mas, vorzugsweise ca. 40 % mas beträgt.

5. Verfahren nach einem der vorhergehenden Ansprüche, **dadurch gekennzeichnet**, daß nach der Hydrolysierung (1) des glutenbildenden und nichtglutenbildenden Proteins sowie nach dem Abbau der nicht protein- und nicht stärkehaltigen Inhaltsstoffe die Trennung der Eiweißfraktion von der Stärke- und Faserfraktion ohne vorhergehende Verdünnung im Zentrifugalfeld (2) vorgenommen wird.

6. Verfahren nach einem der vorhergehenden Ansprüche, **dadurch gekennzeichnet**, daß der überwiegend hydrolysiertes Protein enthaltende Oberlauf (13) aus der Zentrifugation (2) zuerst in einer Eindampfung (6) auf ≧ 40 % mas TS konzentriert und danach auf > 95 % mas TS getrocknet (7) wird.

7. Verfahren nach einem der vorhergehenden Ansprüche, **dadurch gekennzeichnet**, daß als Ausgangsprodukt auch Futterweizen verwendet wird.

8. Verfahren nach einem der vorhergehenden Ansprüche, **dadurch gekennzeichnet**, daß nach der Eindampfung (6) des Proteinhydrolysats ein in einem konventionellen Weizenstärkeprozeß erzeugter Vitalkleber (28) und/oder ein anderes Protein zugegeben wird.

9. Verfahren nach Anspruch 8, **dadurch gekennzeichnet**, daß nach der Zugabe von Vitalkleber (28) und/oder eines anderen Proteins zum Proteinhydrolysat (22) durch Zugabe von Proteasen (32) eine Hydrolysierung des Vitalklebers (28) und/oder eines anderen Proteins vorgenommen wird.

10. Verfahren nach Anspruch 9, **dadurch gekennzeichnet**, daß durch die Menge des zugegebenen Vitalklebers (28) und/oder eines anderen Proteins der Gesamtproteingehalt im Hydrolysat eingestellt wird.

11. Verfahren nach einem der Ansprüche 8 bis 10, **dadurch gekennzeichnet**, daß die Gesamt-TS im Proteinhydrolysat (22) durch Zugabe des Vitalklebers (28) und/oder eines anderen Proteins eingestellt wird.

12. Verfahren nach einem der vorhergehenden Ansprüche, **dadurch gekennzeichnet**, daß aus dem Unterlauf (12) der der Protein-Hydrolysierung (1) nachgeschalteten Zentrifugation (2) eine Stärkehauptfraktion (24) mit stärkehaltigen Fasern sowie eine Restprotein enthaltene Stärkenebenfraktion (14) abgetrennt werden, wobei letztere anschließend in einer kombinierten Fest/Flüssigtrennstufe (4,8) in eine proteinhaltige Phase (20) und in eine stärkehaltige Phase (23) getrennt wird.

13. Verfahren nach Anspruch 12, **dadurch gekennzeichnet**, daß die proteinhaltige Phase (20) mit dem Oberlauf (13) aus der der Protein-Hydrolysierung (1) nachgeschalteten Zentrifugation (2) zusammengeführt wird.

14. Verfahren nach Anspruch 12 oder 13, **dadurch gekennzeichnet**, daß der Oberlauf (10) aus der ersten Trennstufe (4) der kombinierten Fest-/Flüssigtrennstufe (4,8) dem in den Reaktionsbereich (1) zuzuführenden Wasser (33) zugemischt wird.

15. Verfahren nach Anspruch 13, **dadurch gekennzeichnet**, daß auch der Oberlauf (10) aus der ersten Trennstufe (4) der kombinierten Fest-/Flüssigtrennstufe (4,8) mit dem Oberlauf (13) aus der der Protein-Hydrolysierung (1) nachgeschalteten Zentrifugation (2) zusammengeführt wird.

16. Verfahren nach einem der vorhergehenden Ansprüche, dadurch gekennzeichnet, daß zur Erzielung eines säurestabilen Proteinhydrolysats der pH-Wert vor der Eindampfung (6) auf ≦ 5,0 und zur Erzeugung eines nicht säurestabilen Proteinhydrolysats auf > 5,0 eingestellt wird.

17. Vorrichtung zur Durchführung des Verfahrens nach einem der vorhergehenden Ansprüche, **dadurch gekennzeichnet**, daß für das Eintragen der Scherkräfte in die Slurry eine Pumpe, zum Beispiel eine Zentrifugalpumpe mit eingebautem Inline-Sieb, eine Mikrokavikationsaufschlußmaschine oder eine Kolloidmühle vorgesehen ist.

18. Vorrichtung nach Anspruch 17, **dadurch gekennzeichnet**, daß der Pumpe eine Verdrängerpumpe vor- und ein statisches Sieb nachgeschaltet sind.

19. Vorrichtung nach Anspruch 17 oder 18 zur Durchführung des Verfahrens nach Anspruch 12, **dadurch gekennzeichnet**, daß für die der Protein-Hydrolysierung (1) nachgeschaltete Zentrifugation (2) eine Dekanterzentrifuge vorgesehen ist, deren Unterlauf (12) einer zumindest fünf Trennstufen aufweisenden Hydrozyklonanlage (3) zugeführt wird, der für ihren Oberlauf (14) als kombinierte Fest/Flüssigtrennstufe (4,8) ein Separator (4) und diesem für seinen Unterlauf (18) ein Dekanter (8) nachgeschaltet sind.

20. Vorrichtung nach Anspruch 17, 18 oder 19, **dadurch gekennzeichnet**, daß zur Trocknung (7) des eingedampften Proteinhydrolysats (30) ein Sprüh- oder Stromtrockner oder eine Mahl-Trocknungsanlage vorgesehen ist.

21. Proteinhydrolysat, hergestellt nach einem Verfahren nach einem der Ansprüche 1 bis 16 oder auf einer Vorrichtung gemäß einem der Ansprüche 17 bis 20, **dadurch gekennzeichnet**, daß das getrocknete Proteinhydrolysat außer dem Protein weitere Inhaltsstoffe des Weizens oder Weizenmehls wie teilhydrolysierte Pentosane und Glucane, Stärke, Mineralstoffe und niedermolekularen Zucker enthält.

22. Verfahren nach Anspruch 6, **dadurch gekennzeichnet**, daß der Überlauf (13) aus der Zentrifugation (2) zuerst in einem Separator (5) in einen überwiegend hydrolysiertes Protein enthaltenden Oberlauf (16) und einen überwiegend Reststärke enthaltenen Unterlauf (17) getrennt wird.

23. Stärkemilch, hergestellt nach einem Verfahren nach einem der Ansprüche 1 bis 16 oder 22 oder auf einer Vorrichtung gemäß einem der Ansprüche 17 bis 20, **dadurch gekennzeichnet**, daß die unraffinierte Stärkemilch eine Stärkekorngrößenverteilung annähernd der des eingesetzten Weizenmehles aufweist und außer der Stärke weitere Inhaltsstoffe des Weizenmehles wie teilhydrolysierte Pentosane, Glucane, Proteine, Mineralstoffe und niedermolekulare Zucker enthält.

24. Verfahren zur Gewinnung von Weizenstärke und/oder Weizenproteinhydrolysat, **gekennzeichnet durch** folgende Verfahrensschritte:
a) als Ausgangsstoffe werden bei der Weizenstärkegewinnung anfallende Zwischen- und gegebenenfalls zusätzlich Endprodukte verwendet;
b) aus den Ausgangsstoffen wird eine Slurry hergestellt, die auf eine Temperatur ≦ 50 °C eingestellt wird;
c) dieser Slurry werden Enzyme zugegeben, um das glutenbildende und nicht glutenbildende Weizenprotein sowie weitere nicht protein- und nicht stärkehaltige Inhaltsstoffe in der Slurry so zu hydrolysieren, daß einhergehend mit der Erniedrigung der Viskosität in der Slurry die Stärkekörner von den Nichtstärkekomponenten freigesetzt werden;
d) die Slurry wird auf die gewünschte Temperatur sowie auf einen pH-Wert > 4,0 und < 7,0 eingestellt;
e) in die Mischung werden Scherkräfte eingetragen, und zwar durch Umpumpen der Mischung durch ein Sieb, wobei den Feststoffanteilen der Mischung unmittelbar vor dem Sieb mittels einer eingesetzten Pumpe eine hohe Beschleunigung erteilt wird.

25. Verfahren nach Anspruch 24, **dadurch gekennzeichnet**, daß als Endprodukt nicht getrockneter und/oder getrockneter Weizenkleber und/oder andere Proteinquellen verwendet werden.

26. Verfahren nach Anspruch 24 oder 25, **dadurch gekennzeichnet**, daß als Ausgangsstoff zusätzlich Weizenmehl verwendet wird.

27. Verfahren nach Anspruch 26, **dadurch gekennzeichnet**, daß die Slurry nach der Enzymreaktion und einer Fasersiebung durch Trennung in einem Zentrifugalfeld getrennt wird in eine Stärke enthaltende Fraktion und in eine Proteinfraktion.

28. Verfahren nach Anspruch 27, **dadurch gekennzeichnet**, daß zur Trennung im Zentrifugalfeld ein Dekanter, Separator oder Hydronzyklon verwendet wird.

29. Verfahren nach einem der Ansprüche 25 bis 28, **dadurch gekennzeichnet**, daß durch Zugabe von Prozeßwasser der TS-Gehalt in der Slurry für die Einwirkung der Enzyme auf ≧ 10 % mas, vorzugsweise ca. 40 % mas, eingestellt wird.

30. Verfahren nach einem der Ansprüche 24 - 29, **dadurch gekennzeichnet**, daß für die Hydrolysierung des glutenbildenden und nicht glutenbildenden Proteins Proteasen und Peptidasen, für den Abbau von nicht protein- und nicht stärkehaltigen Inhaltsstoffen, Hemicellulasen, Pentosanasen, Cellulasen, Glucanasen, Alpha-Galactosidasen, Pektinasen, Xylanasen, Phospholipasen, Arabinasen, Cellobiasen oder eine Mischung derselben zugegeben werden.

31. Verfahren nach einem der Ansprüche 24 - 30, **gekennzeichnet durch** die Verwendung der nach der Kleberabtrennung als Zwischenprodukt anfallenden B-Stärke gemeinsam mit der als Zwischenprodukt anfallenden C-Stärke.

32. Verfahren nach einem der Ansprüche 24 bis 30, **dadurch gekennzeichnet**, daß die nach der Kleberabtrennung als Zwischenprodukt anfallende B-Stärke sowie die als Zwischenprodukt anfallende C-Stärke getrennt der jeweiligen Enzymreaktionen zugeführt werden.

33. Verfahren nach Anspruch 31 oder 32, **dadurch gekennzeichnet**, daß vor der Verwendung der B-Stärke eine Klebersiebung und gegebenenfalls eine A-Stärke-Rückgewinnung durchgeführt werden.

34. Verfahren nach Anspruch 31, 32 oder 33, **dadurch gekennzeichnet**, daß vor der Verwendung der C-Stärke eine Klebersiebung durchgeführt wird.

35. Verfahren nach einem der Ansprüche 24 - 34, **dadurch gekennzeichnet**, daß der als Endprodukt verwendete getrocknete und/oder nicht getrocknete Weizenkleber und/oder andere Proteinquellen gemeinsam mit Weizenmehl als Ausgangsstoff den Zwischenprodukten zugesetzt werden, um definierte Stärke- und/oder Proteinkonzentrationen im Weizenproteinhydrolysat einzustellen.

36. Verfahren nach einem der Ansprüche 24 - 35, **dadurch gekennzeichnet**, daß die nach der Trennung im Zentrifugalfeld enthaltene Proteinfraktion in einer Eindampfanlage konzentriert wird.

37. Verfahren nach Anspruch 36, **dadurch gekennzeichnet**, daß zur Trocknung der eingedampften Proteinfraktion ein Sprüh- oder Stromtrockner oder eine Mahltrocknungsanlage verwendet wird.

38. Verfahren nach einem der Ansprüche 24 bis 31 und 33 bis 37, **dadurch gekennzeichnet**, daß der als Endprodukt verwendete getrocknete und/oder nicht getrocknete Weizenkleber und/oder andere Proteinquellen mit Weizenmehl als Ausgangsstoff gemeinsam einer Reaktion zugeführt werden und das erhaltene Reaktionsgemisch mit der eingedampften Proteinfraktion zusammengeführt wird.

39. Verfahren zur Gewinnung von Weizenproteinhydrolysat aus Weizen, geschältem Weizen, Weizenmehl oder proteinangereichertem Weizenmehl, wobei der Weizen oder geschälte Weizen zuerst trocken oder naß vermahlen wird, das Weizenmehl oder protein-angereicherte Weizenmehl ausschließlich naß vermahlen werden kann und danach mit Wasser bei Temperaturen ≦ 50°C zu einer Slurry verarbeitet wird, die anschließend durch Zentrifugieren getrennt wird in eine Stärke und Fasern enthaltende Fraktion und in eine glutenbildende Proteinfraktion, **gekennzeichnet durch** folgende Verfahrensschritte:
a) nach dem Zentrifugieren werden der glutenbildenden Proteinfraktion Enzyme zugegeben, um das glutenbildende und nicht glutenbildende Weizenprotein sowie weitere nicht protein- und nicht stärkehaltige Inhaltsstoffe in der glutenbildenden Proteinfraktion zu einem Weizenproteinhydrolysat zu hydrolysieren;
b) die Slurry wird auf die gewünschte Temperatur sowie auf einen pH-Wert > 4,0 und < 7,0 eingestellt;
c) in die Mischung werden Scherkräfte eingetragen und zwar durch Umpumpen der Mischung durch ein Sieb, wobei den Feststoffanteilen der Mischung unmittelbar vor dem Sieb mittels einer eingesetzten Pumpe eine hohe Beschleunigung erteilt wird.

40. Verfahren nach einem der Ansprüche 1 bis 16, 22 und 24 bis 39, **dadurch gekennzeichnet,** daß zur Inaktivierung der eingesetzten Enzyme ein pH-Wert von ≧ 4,0 und ≦ 6,0 sowie eine Temperatur von ≦ 85 °C in dem Konzentrat vor der abschließenden Trocknung für länger als 10 Minuten eingestellt werden.
